# EUROPEAN PATENT SPECIFICATION

(11) **EP 1 430 139 B1**
(45) Date of publication and mention of the grant of the patent: **10.05.2006**
(21) Application number: 02760324.0
(22) Date of filing: 22.08.2002
(51) Int. Cl.: C12P 13/02, C12P 7/42, C12N 15/31, A23K 1/16

(54) **PROCESS FOR THE PREPARATION OF D-PANTOTHENIC ACID AND/OR ITS SALTS**
VERFAHREN ZUR HERSTELLUNG VON D-PANTOTHENSÄURE UND/ODER IHREN SALZEN
PROCEDE DE PREPARATION D'ACIDE D-PANTOTHENIQUE ET/OU DE SELS DE CE DERNIER

(30) Priority: 28.09.2001 DE 10147960
(43) Date of publication of application: 23.06.2004
(73) Proprietor: Degussa AG, 40474 Düsseldorf (DE)
(72) Inventor: RIEPING, Mechthild, 33619 Bielefeld (DE)
(86) International application number: PCT/EP2002/009373
(87) International publication number: WO 2003/029476

(56) References cited:
- EP-A- 0 590 857
- DE-A- 10 130 192
- US-B1- 6 171 845
- US-B1- 6 184 007
- YUN M ET AL: "Structural basis for the feedback regulation of Escherichia coli pantothenate kinase by coenzyme A." THE JOURNAL OF BIOLOGICAL CHEMISTRY, vol. 275, no. 36, 8 September 2000 (2000-09-08), pages 28093-28099, XP002235164 US ISSN: 0021-9258

## Description

### Field of the Invention

The present invention relates to a process for the preparation of D-pantothenic acid and/or its salts or mixtures containing them using microorganisms of the family Enterobacteriaceae in which at least the ytfP and/or yjfA open reading frame (ORF) is attenuated, by mutation.

### Prior Art

Pantothenic acid is produced throughout the world on a scale of several thousand tons per year. It is used inter alia in human medicine, in the pharmaceutical industry and in the food industry. A large part of the pantothenic acid produced is used for feeding farm animals such as poultry and pigs.

Pantothenic acid can be prepared by chemical synthesis or biotechnologically by the fermentation of suitable microorganisms in suitable nutrient solutions. In the chemical synthesis, DL-pantolactone is an important precursor. It is prepared in a multistage process from formaldehyde, isobutyl aldehyde and cyanide. In subsequent process steps the racemic mixture is separated and the D-pantolactone is condensed with β-alanine to give D-pantothenic acid.

The typical commercial form is the calcium salt of D-pantothenic acid. The calcium salt of the racemic mixture of DL-pantothenic acid is also useful.

The advantage of preparation by the fermentation of microorganisms is the direct formation of the desired stereoisomeric form, namely the D form, free of L-pantothenic acid.

As indicated in EP-A 0 493 060, various species of bacteria, e.g. Escherichia coli (E. coli), Arthrobacter ureafaciens, Corynebacterium erythrogenes and Brevibacterium ammoniagenes, as well as yeasts, e.g. Debaromyces castellii, can produce D-pantothenic acid in a nutrient solution containing glucose, DL-pantoic acid and β-alanine. EP-A 0 493 060 further indicates that, in the case of E. coli, the formation of D-pantothenic acid is improved in a nutrient solution containing glucose, DL-pantoic acid and β-alanine by amplification of the pantothenic acid biosynthesis genes from E. coli which are contained on plasmids pFV3 and pFV5.

EP-A 0 590 857 and US patent 5,518,906 describe mutants derived from the E. coli strain IFO3547, such as FV5714, FV525, FV814, FV521, FV221, FV6051 and FV5069, which carry resistances to various antimetabolites such as salicylic acid, α-ketobutyric acid, β-hydroxyaspartic acid, O-methylthreonine and α-ketoisovaleric acid. They produce pantoic acid in a nutrient solution containing glucose, and D-pantothenic acid in a nutrient solution containing glucose and β-alanine. EP-A 0 590 857 and US patent 5,518,906 further indicate that, after amplification of the pantothenic acid biosynthesis genes panB, panC and panD, which are said to be contained on plasmid pFV31, in the above-mentioned strains, the production of D-pantoic acid is improved in nutrient solutions containing glucose and the production of D-pantothenic acid is improved in a nutrient solution containing glucose and β-alanine.

Furthermore, WO97/10340 reports the beneficial effect of amplification of the ilvGM operon on the production of D-pantothenic acid. Finally, EP-A 1 001 027 reports the effect of amplification of the panE gene on the formation of D-pantothenic acid.

The D-pantothenic acid or the corresponding salt is isolated from the fermentation broth and purified as in the prior art (EP-A 0 590 857 and WO96/33283) and accordingly used in purified form, or the whole of the fermentation broth containing the D-pantothenic acid is dried (EP-A 1 050 219) and used in particular as an animal feed additive.

### Object of the Invention

The inventors set out to provide novel measures to improve the preparation, by fermentation, of D-pantothenic acid and/or its salts or animal feed additives containing them.

### Summary of the Invention

Whenever D-pantothenic acid, pantothenic acid or pantothenate is mentioned hereafter, this is understood as meaning not only the free acids but also the salts of D-pantothenic acid, e.g. the calcium, sodium, ammonium or potassium salt.

The invention provides a process for the preparation of D-pantothenic acid and/or its salts or animal feed additives obtaining them by the fermentation of genetically modified microorganisms of the family Enterobacteriaceae, especially those which already produce D-pantothenic acid, whereby the activity or concentration of the proteins encoded by the ytfP open reading frame having the nucleotide sequence of nucleotides 376 to 714 of SEQ ID NO:1 and the yjfA open reading frame having the nucleotide sequence of nucleotides 727 to 461 of the complement of SEQ ID NO:1 is reduced to 0 % of the activity or concentration of the corresponding proteins in the starting microorganisms.

The process is optionally carried out in the presence of alkaline earth metal compounds introduced continuously or batchwise, preferably in stoichiometric amounts,
c) the D-pantothenic acid or the corresponding salt is enriched in the medium or the fermentation broth or in the cells of the microorganisms of the family Enterobacteriaceae, and
d) when the fermentation has finished, the D-pantothenic acid and/or the corresponding salt are isolated.

The invention also provides a process in which, when the fermentation has finished, all or part of the biomass (≥0 to 100%) is separated from the fermentation broth, or optionally remains in it, and the resulting broth is processed, optionally after concentration, to a solid mixture containing D-pantothenic acid and/or its salts together with other constituents of the fermentation broth.

The invention also provides genetically modified microorganisms of the family Enterobacteriaceae, especially of the genus Escherichia, in which the ytfP ORF and/or yifA ORF or their gene products are not present.

### Detailed Description of the Invention

In general the term 'attenuation' describes the reduction or switching-off, in a microorganism, of the intracellular activity of one or more enzymes or proteins coded for by the appropriate DNA, for example by using a weak promoter or using a gene or allele or an open reading frame (ORF) which codes for an appropriate enzyme or protein with a low activity or inactivates the appropriate gene, ORF or enzyme or protein, and optionally combining these measures.

An open reading frame (ORF) is a segment of a nucleotide sequence which codes or can code for a protein or polypeptide or a ribonucleic acid to which no function can be assigned according to the prior art. After a function has been assigned to the particular segment of the nucleotide sequence, the term 'gene' is generally used.

The attenuation measures of the invention, including the reduction of expression, reduce the activity or concentration of the appropriate protein to 0% of the activity or concentration of the protein in the starting microorganism.

The microorganisms provided by the present invention can produce D-pantothenic acid from glucose, sucrose, lactose, fructose, maltose, molasses, starch and cellulose or from glycerol and ethanol. They are representatives of the Enterobacteriaceae, especially of the genus Escherichia. The species Escherichia coli may be mentioned in particular within the genus Escherichia. Suitable strains within the species Escherichia coli are the so-called K-12 strains, e.g. the strains MG1655 or W3110 (Neidhard et al.: Escherichia coli and Salmonella. Cellular and Molecular Biology (ASM Press, Washington DC)) or the Escherichia coli wild-type strain IFO3547 (Institute of Fermentation, Osaka, Japan) and mutants derived therefrom which are capable of producing D-pantothenic acid.

Examples of suitable D-pantothenic acid-producing strains of the genus Escherichia, especially of the species Escherichia coli, are:
Escherichia coli FV5069/pFV31
Escherichia coli FV5069/pFV202
Escherichia coli FE6/pFE80 and
Escherichia coli KE3

It has been found that the production of D-pantothenic acid by Enterobacteriaceae is improved after attenuation of the ytfP and/or yjfA open reading frames according to the measures of the invention, whereby activity and concentration of the corresponding proteins are reduced to 0 % of the activity or concentration of the starting microorganisms.

The nucleotide sequence of the ytfP ORF is published under Accession Number AAC77179 and the nucleotide sequence of the yjfA ORF is published under Accession Number AAC77180 at the National Center for Biotechnology Information (NCBI, Bethesda, MD, USA). They can also be taken from the genome sequence of Escherichia coli published by Blattner et al. (Science 277, 1453 - 1462 (1997)).

The open reading frames described in the cited literature references can be used according to the invention. It is also possible to use alleles which result from the degeneracy of the genetic code or from neutral sense mutations.

Attenuation in general can be achieved for example by reducing or switching off the expression of the ytfP ORF and/or yjfA ORF or the catalytic properties of the protein. Both measures may optionally be combined.

Reduction of the gene expression can be achieved by an appropriate culture technique, by genetic modification (mutation) of the signal structures of gene expression or by antisense RNA technology. Examples of signal structures of gene expression are repressor genes, activator genes, operators, promoters, attenuators, ribosome binding sites, the start codon and terminators. Those skilled in the art can find relevant details inter alia in e.g. Jensen and Hammer (Biotechnology and Bioengineering 58: 191 - 195 (1998)), Carrier and Keasling (Biotechnology Progress 15; 58 - 64 (1999)), Franch and Gerdes (Current Opinion in Microbiology 3, 159 - 164 (2000)) and well-known textbooks on genetics and molecular biology, for example the textbook by Knippers ("Molekulare Genetik", 6th edition, Georg Thieme Verlag, Stuttgart, Germany, 1995) or the textbook by Winnacker ("From Genes to Clones", VCH Verlagsgesellschaft, Weinheim, Germany, 1990).

Mutations which lead to a change or reduction of the catalytic properties of enzyme proteins are known from the prior art. Examples which may be mentioned are the studies by Qiu and Goodman (Journal of Biological Chemistry 272: 8611 - 8617 (1997)), Yano et al. (Proceedings of the National Academy of Sciences, USA 95, 5511 - 5515 (1998)) and Wente and Schachmann (Journal of Biological Chemistry 266, 20833 - 20839 (1991)). Surveys can be found in well-known textbooks on genetics and molecular biology, e.g. the textbook by Hagemann ("Allgemeine Genetik", Gustav Fischer Verlag, Stuttgart, 1986).

Possible mutations according to the invention are transitions, transversions, insertions and deletions. Depending on the effect of amino acid exchange on enzyme activity, the term 'missense mutations; or 'nonsense mutations' is used. Insertions or deletions of at least one base pair in a gene lead to frame shift mutations, the effect of which is to incorporate false amino acids or terminate the translation prematurely. Deletions of several codons typically result in a complete disappearance of enzyme activity. Instructions on the production of such mutations belong to the prior art and can be found in well-known textbooks on genetics and molecular biology, e.g. the textbook by Knippers ("Molekulare Genetik", 6th edition, Georg Thieme Verlag, Stuttgart, Germany, 1995), the textbook by Winnacker ("From Genes to Clones", VCH Verlagsgesellschaft, Weinheim, Germany, 1990) or the textbook by Hagemann ("Allgemeine Genetik", Gustav Fischer Verlag, Stuttgart, 1986).

Suitable mutations in the ytfP ORF and/or yjfA ORF, for example deletion mutations, can be incorporated into suitable strains by gene or allele exchange.

A common method is that of gene exchange using a conditionally replicating pSC101 derivative, pMAK705, as described by Hamilton et al. (Journal of Bacteriology 171, 4617 - 4622 (1989)). Other methods described in the prior art, for example that of Martinez-Morales et al. (Journal of Bacteriology 181, 7143 - 7148 (1999)) or that of Boyd et al. (Journal of Bacteriology 182, 842 - 847 (2000)), can likewise be used.

Also, mutations in the ytfP ORF and/or yjfA ORF or mutations which affect the expression of the ytfP ORF and/or yjfA ORF can be transferred to different strains by conjugation or transduction.

Furthermore, for the production of D-pantothenic acid with strains of the family Enterobacteriaceae, it can be advantageous not only to delete the ytfp ORF and/or yjfA ORF, but also to amplify or, in particular, overexpress one or more endogenous genes, i.e. genes inherent in the particular species, selected from the group comprising:
- the ilvGM operon coding for acetohydroxy acid synthase II (WO97/10340),
- the panB gene coding for ketopantoate hydroxymethyltransferase (US-A-5,518,906),
- the panE gene coding for ketopantoate reductase (EP-A-1 001 027),
- the panD gene coding for aspartate decarboxylase (US-A-5,518,906),
- the panC gene coding for pantothenate synthetase (US-A-5,518,906),
- the serC gene coding for phosphoserine transaminase (Duncan and Coggins, Biochemical Journal 234: 49 - 57 (1986)),
- the gcvT, gcvH and gcvP genes coding for the glycine cleavage system (Okamura-Ikeda et al., European Journal of Biochemistry 216, 539 - 548 (1993)), and
- the glyA gene coding for serine hydroxymethyltransferase (Plamann et al. (Nucleic Acids Research 11(7): 2065 - 2075 (1983))).

In this context the term 'amplification' describes the increase, in a microorganism, of the intracellular activity of one or more enzymes or proteins coded for by the appropriate DNA, for example by increasing the copy number of the gene or genes, using a strong promoter or a gene or allele coding for an appropriate enzyme or protein with a high activity, and optionally combining these measures.

Through the measures of amplification, especially overexpression, the activity or concentration of the appropriate protein is generally increased at least by 10%, 25%, 50%, 75%, 100%, 150%, 200%, 300%, 400% or 500%, and at most by up to 1000% or 2000%, based on that of the wild-type protein or the activity or concentration of the protein in the starting microorganism.

Finally, for the production of D-pantothenic acid with strains of the family Enterobacteriaceae, it can be advantageous not only to attenuate the ytfP ORF and/or yjfA ORF, but also to attenuate individually or together:
- the avtA gene coding for transaminase C (EP-A-1 001 027),
- the poxB gene coding for pyruvate oxidase (Grabau and Cronan, Nucleic Acids Research 14 (13), 5449 - 5460 (1986)), and
- the pckA gene coding for PEP carboxykinase (Medina et al., Journal of Bacteriology 172, 7151 - 7156 (1990)).

Furthermore, for the production of D-pantothenic acid, it can be advantageous not only to attenuate the ytfP ORF and/or yjfA ORF, but also to switch off unwanted secondary reactions (Nakayama: "Breeding of Amino Acid Producing Microorganisms", in: Overproduction of Microbial Products, Krumphanzl, Sikyta, Vanek (eds.), Academic Press, London, UK , 1982).

The microorganisms prepared according to the invention can be cultivated by the batch process, the fed batch process or the repeated fed batch process. A summary of known cultivation methods is provided in the textbook by Chmiel (Bioprozesstechnik 1. Einführung in die Bioverfahrenstechnik (Gustav Fischer Verlagr, Stuttgart, 1991)) or in the textbook by Storhas (Bioreaktoren und periphere Einrichtungen (Vieweg Verlag, Brunswick/Wiesbaden, 1994)).

The culture medium to be used must appropriately meet the demands of the particular strains. Descriptions of culture media for various microorganisms can be found in "Manual of Methods for General Bacteriology" of the American Society for Bacteriology (Washington DC, USA, 1981). Carbon sources which can be used are sugars and carbohydrates, e.g. glucose, sucrose, lactose, fructose, maltose, molasses, starch and cellulose, oils and fats, e.g. soya bean oil, sunflower oil, groundnut oil and coconut fat, fatty acids, e.g. palmitic acid, stearic acid and linoleic acid, alcohols, e.g. glycerol and ethanol, and organic acids, e.g. acetic acid. These substances can be used individually or as a mixture.

Nitrogen sources which can be used are organic nitrogen-containing compounds such as peptones, yeast extract, meat extract, malt extract, corn steep liquor, soya bean flour and urea, or inorganic compounds such as ammonium sulfate, ammonium chloride, ammonium phosphate, ammonium carbonate and ammonium nitrate. The nitrogen sources can be used individually or as a mixture.

Phosphorus sources which can be used are phosphoric acid, potassium dihydrogenphosphate or dipotassium hydrogenphosphate or the corresponding sodium salts. The culture medium must also contain metal salts, e.g. magnesium sulfate or iron sulfate, which are necessary for growth. Finally, essential growth-promoting substances such as amino acids and vitamins can be used in addition to the substances mentioned above. D-pantothenic acid precursors, such as aspartate, β-alanine, ketoisovalerate, ketopantoic acid or pantoic acid, and optionally their salts, can also be added to the culture medium. Said feed materials can be added to the culture all at once or fed in appropriately during cultivation.

The pH of the culture is controlled by the appropriate use of basic compounds such as sodium hydroxide, potassium hydroxide, ammonia or aqueous ammonia, or acid compounds such as phosphoric acid or sulfuric acid.

As a further possibility, to prepare the alkaline earth metals salts of pantothenic acid, especially the calcium salt, a suspension or solution of an inorganic compound containing an alkaline earth metal, for example calcium hydroxide, or of an organic compound such as an alkaline earth metal salt of an organic acid, for example calcium acetate, is added continuously or batchwise during the fermentation. In this way the cation required to prepare the desired alkaline earth metal salt of D-pantothenic acid is introduced into the fermentation broth directly in the desired amount, generally in a proportion of 0.8 to 1.2, based on the pantothenic acid, and preferably in stoichiometric amounts.

Foaming can be controlled using antifoams such as fatty acid polyglycol esters. The stability of plasmids can be maintained by adding suitable selectively acting substances, e.g. antibiotics, to the medium. Aerobic conditions are maintained by introducing oxygen or oxygen-containing gaseous mixtures, e.g. air, into the culture. The temperature of the culture is normally 25ºC to 45ºC and preferably 30ºC to 40ºC. The culture is continued until the formation of D-pantothenic acid has reached a maximum. This objective is normally achieved within 10 hours to 160 hours.

The D-pantothenic acid or corresponding D-pantothenic acid salts contained in the fermentation broth can then be isolated and purified according to the prior art.

It is also possible to remove all or part (≥0 to 100%) of the biomass from the fermentation broths containing D-pantothenic acid and/or its salts, preferably initially by means of known methods of separation, for example centrifugation, filtration, decantation or a combination thereof. Another possibility, however, is to leave the whole of the biomass in the fermentation broth.

In general the suspension or solution is preferably concentrated and processed to a powder, for example using a spray dryer or a freeze-drying unit. This powder is then generally converted by means of suitable compaction or granulation processes, e.g. pelletizing, to a coarser, easily flowable, storable and largely dust-free product with the desired particle size distribution of optionally 20 to 2000 µm, especially of 100 to 1400 µm. In the granulation or compaction process it is advantageous to use conventional organic or inorganic auxiliary substances or carriers like starch, gelatin, cellulose derivatives or similar substances, such as those conventionally used as binders, gelling agents or thickeners in food or animal feed processing, or to use other substances like silicic acids, silicates or stearates.

Alternatively, the fermentation product, with or without other conventional constituents of the fermentation broth, can be absorbed onto an organic or inorganic carrier known and conventionally used in animal feed processing, for example silicic acids, silicates, meals, brans, flours, starches, sugars or the like, and/or stabilized with conventional thickeners or binders. Relevant application examples and processes are described in the literature (Die Mühle + Mischfuttertechnik 132 (1995) 49, page 817).

Optionally, at a suitable stage of the process, D-pantothenic acid, the desired D-pantothenic acid salt or a preparation containing these compounds is added in order to attain the desired content of pantothenic acid or the desired salt, or adjust said content to the desired value, in the end product.

The desired content generally ranges from 20 to 80 wt.% (dry weight).

The concentration of pantothenic acid can be determined by known chemical methods (Velisek; Chromatographic Science 60, 515 - 560 (1992)) or microbiological methods, e.g. the Lactobacillus plantarum test (DIFCO MANUAL, 10th edition, pp 1100 - 1102; Michigan, USA).

A pure culture of the Escherichia coli K-12 strain DH5α/pMAK705 was deposited as DSM 13720 on 08 September 2000 in the Deutsche Sammlung für Mikroorganismen und Zellkulturen (DSMZ, Brunswick, Germany) under the terms of the Budapest Treaty.

A pure culture of the Escherichia coli K-12 strain FE6-1 was deposited as DSM 13721 on 08 September 2000 in the Deutsche Sammlung fur Mikroorganismen und zellkulturen (DSMZ, Brunswick, Germany) under the terms of the Budapest Treaty,

The present invention is illustrated in greater detail below with the aid of embodiment examples.

The minimum medium (M9) and complete medium (LB) used for Escherichia coli are described by J.H. Miller (A Short Course In Bacterial Genetics (1992), Cold Spring Harbor Laboratory Press). The isolation of plasmid DNA from Escherichia coli, as well as all the techniques for restriction, ligation, Klenow treatment and alkaline phosphatase treatment, are performed according to Sambrook et al. (Molecular Cloning - A Laboratory Manual (1989) Cold Spring Harbor Laboratory Press). Unless described otherwise, the transformation of Escherichia coli is performed according to Chung et al. (Proceedings of the National Academy of Sciences of the United States of America, USA (1989) 86: 2172 - 2175).

The incubation temperature in the preparation of strains and transformants is 37°C. Temperatures of 30°C and 44°C are used in the gene exchange process according to Hamilton et al.

### Example 1

### Construction of the deletion mutation of the ytfP-yjfA gene region

The ytfP-yjfA gene region is amplified from Escherichia coli K12 using the polymerase chain reaction (PCR) and synthetic oligonucleotides. The following PCR primers (MWG Biotech, Ebersberg, Germany) are synthesized from the nucleotide sequence of the ytfP-yjfA gene region in E. coli K12 MG1655 (SEQ ID No. 1):

The chromosomal E. coli K12 MG1655 DNA used for the PCR is isolated with "Qiagen Genomic-tips 100/G" (QIAGEN, Hilden, Germany) according to the manufacturer's instructions. An approx. 1250 bp DNA fragment can be amplified with the specific primers under standard PCR conditions (Innis et al. (1990) PCR Protocols. A Guide to Methods and Applications, Academic Press) using Taq DNA polymerase (Gibco-BRL, Eggenstein, Germany). The PCR product is ligated with vector pCR2. 1TOPO (TOPO TA Cloning Kit, Invitrogen, Groningen, The Netherlands) according to the manufacturer's instructions and transformed into the E. coli strain TOP10F'. Plasmid-carrying cells are selected on LB agar to which 50 µg/ml of ampicillin have been added. After isolation of the plasmid DNA, the successful cloning of the PCR product is checked with the restriction enzymes EcoRI and NsiI.

To produce a 337 bp deletion in the ytfP-yjfA region, vector pCR2.1TOPOytfP-yjfA is cleaved with the restriction enzymes NdeI and SspI and the 4.8 kbp DNA fragment is treated with the Klenow enzyme and then ligated.

The E. coli strain DH5α is transformed with the ligation mixture and plasmid-carrying cells are selected on LB agar to which 50 µg/ml of ampicillin have been added. After isolation of the plasmid DNA, plasmids in which the mutagenic DNA sequence shown in SEQ ID No. 4 is present in cloned form are detected by control cleavage with the enzyme EcoRI. The plasmid is called pCR2.ITOPOΔyjfA.

### Example 2

### Construction of exchange vector pMAK705ΔyjfA

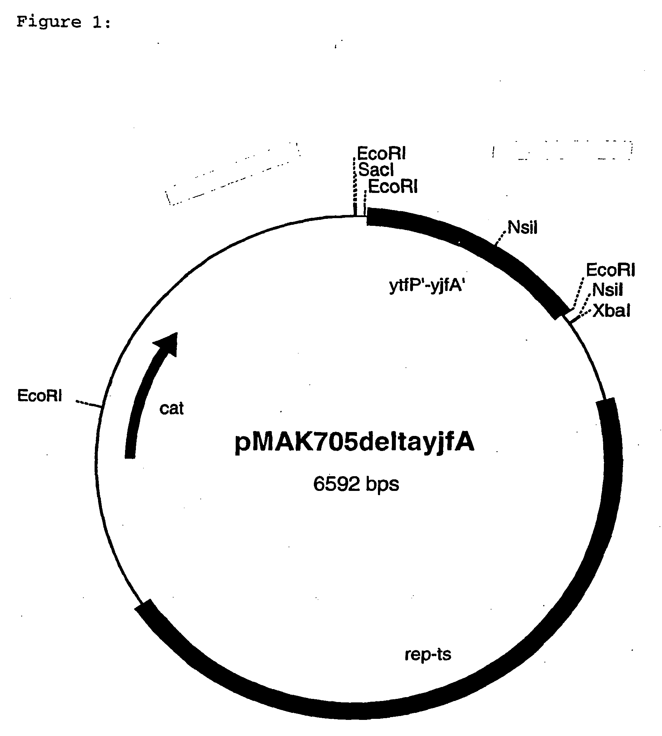

The ytfP-yjfA allele described in Example 1 is isolated from vector pCR2.1TOPOΔyjfA after restriction with the enzymes SacI and XbaI and separation in 0.8% agarose gel, and ligated with plasmid pMAK705 (Hamilton et al. (1989) Journal of Bacteriology 171, 4617 - 4622) digested with the enzymes SacI and XbaI. The ligation mixture is transformed into DH5α and plasmid-carrying cells are selected on LB agar to which 20 µg/ml of chloramphenicol have been added. The successful cloning is detected after isolation of the plasmid DNA and cleavage with the enzymes SacI and XbaI. The exchange vector formed, pMAK705AyjfA (= pMAK705deltayjfA), is shown in Figure 1.

### Example 3

Site-specific mutagenesis of the ytfP-yjfA gene region in the E. coli strain FE6-1

The E. coli strain FE6 is a valine-resistant mutant of E. coli K12 MG1655 (US-B-6171845) and is deposited as DSM12379 in the Deutsche Sammlung für Mikroorganismen und Zellkulturen (DSMZ, Brunswick, Germany). Spontaneous mutants are isolated from FE6 after incubation at 37°C on minimal agar to which 2 g/l of glucose and 1 g/l of β-hydroxyaspartic acid have been added.

A chosen β-hydroxyaspartic acid-resistant single colony is then incubated at 37°C on minimal agar containing 2 g/l of glucose and 0.2 g/l of O-methylthreonine. After this step, a mutant called FE6-1 is resistant to valine, α-ketoisovaleric acid, β-hydroxyaspartic acid and O-methylthreonine. For exchange of the chromosomal ytfP-yjfA gene region with the plasmid-coded deletion construct, FE6-1 is transformed with plasmid pMAK705ΔyjfA. The gene exchange is effected by the selection method described by Hamilton et al. (1989) Journal of Bacteriology 171, 4617 - 4622, and is verified by standard PCR methods (Innis et al. (1990) PCR Protocols. A Guide to Methods and Applications, Academic Press) using the following oligonucleotide primers:

The strain obtained is called FE6-1ΔyjfA.

### Example 4

### Preparation of D-pantothenic acid with the strain FE6-1ΔyjfA/pFV31ilvGM

### 4.1 Amplification and cloning of the ilvGM gene

The ilvGM operon from Escherichia coli IFO3547 coding for acetohydroxy acid synthase II (Institute of Fermentation, Osaka, Japan) is amplified using the polymerase chain reaction (PCR) and synthetic oligonucleotides. PCR primers (MWG Biotech, Ebersberg, Germany) are synthesized from the nucleotide sequence of the ilvGM operon in E. coli K12 MG1655 (GenBank: Accession No. M87049). The sequence of the ilvGM1 primer is chosen so that the primer contains an adenine in position 8. This generates a modified ribosome binding site 7 nucleotides upstream from the start codon of the ilvG proteins.

The chromosomal E. coli IFO3547 DNA used for the PCR is isolated with "QIAGEN Genomic-tips 100/G" (QIAGEN, Hilden, Germany) according to the manufacturer's instructions. An approx. 2100 bp DNA fragment comprising the modified ribosome binding site, the ilvGM coding regions and approx. 180 bp 3'-flanking sequences can be amplified with the specific primers under standard PCR conditions (Innis et al.: PCR Protocols. A Guide to Methods and Applications, 1990, Academic Press) using Pfu DNA polymerase (Promega Corporation, Madison, USA). The PCR product is cloned into plasmid pCR-BluntII-TOPO and transformed into the E. coli strain TOP10 (Invitrogen, Groningen, The Netherlands, product description Zero Blunt TOPO PCR Cloning Kit, Cat. No. K2800-20).

The successful cloning is detected by cleavage of plasmid pCR-BluntIF03547ilvGM with the restriction enzymes EcoRI and SphI. To do this, the plasmid DNA is isolated by means of the "QIAprep Spin Plasmid Kit" (QIAGEN, Hilden, Germany), cleaved and then separated in a 0.8% agarose gel. The DNA sequence of the amplified fragment is determined using the reverse and universal sequencing primers (QIAGEN, Hilden, Germany). The sequence of the PCR product is shown in SEQ ID No. 7 and 9. The ilvG gene or allele is identified in SEQ ID No. 7. The ilvM gene or allele is identified in SEQ ID No. 9. The corresponding gene products or proteins are shown in SEQ ID No. 8 and 10.

### 4.2 Cloning of the ilvGM genes into expression vector pTrc99A

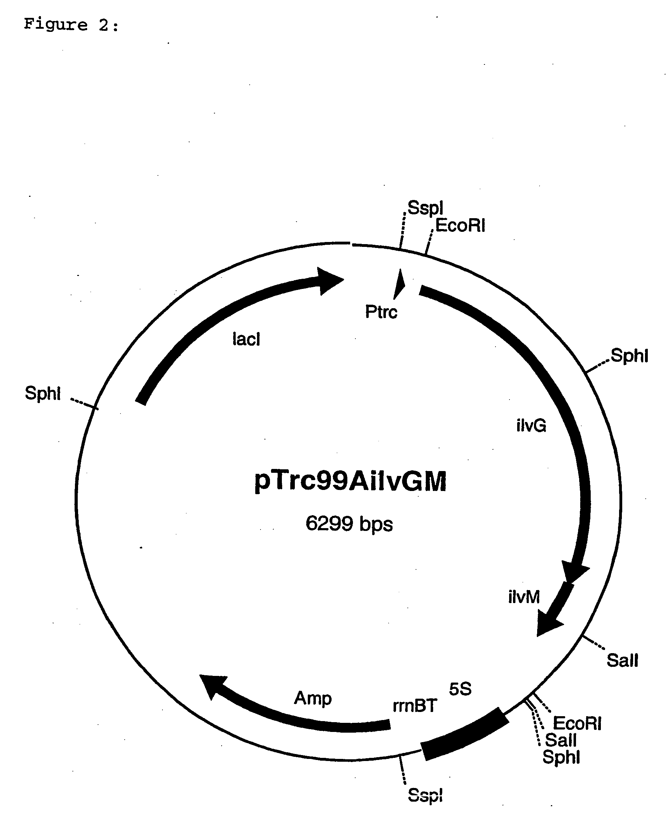

For expression in Escherichia coli K12, the ilvGM genes described in Example 4.1 are cloned into vector pTrc99A (Amersham Pharmacia Biotech Inc., Uppsala, Sweden). This is done by cleaving plasmid pCR-BluntIFO3547ilvGM with the enzyme EcoRI, separating the cleavage mixture in 0.8% agarose gel and isolating the 2.1 kbp ilvGM fragment using the QIAquick Gel Extraction Kit (QIAGEN, Hilden, Germany). Vector pTrc99A is cleaved with the enzyme EcoRI, an alkaline phosphatase treatment is carried out and the product is ligated with the isolated ilvGM fragment. The ligation mixture is transformed into the E. coli strain DH5α. pTrc99A-carrying cells are selected on LB agar (Lennox, Virology 1: 190 (1955)) to which 50 µg/ml of ampicillin have been added. The successful cloning of the ilvGM operon can be detected after isolation of the plasmid DNA and control cleavage with SalI and SphI. In the vector called pTrc99AilvGM (Figure 2), expression of the ilvGM operon is regulated by the Ptrc promoter located upstream from the modified ribosome binding site and by the rRNA terminator region located downstream from the ilvGM coding regions.

### 4.3 Construction of vector pFV31ilvGM

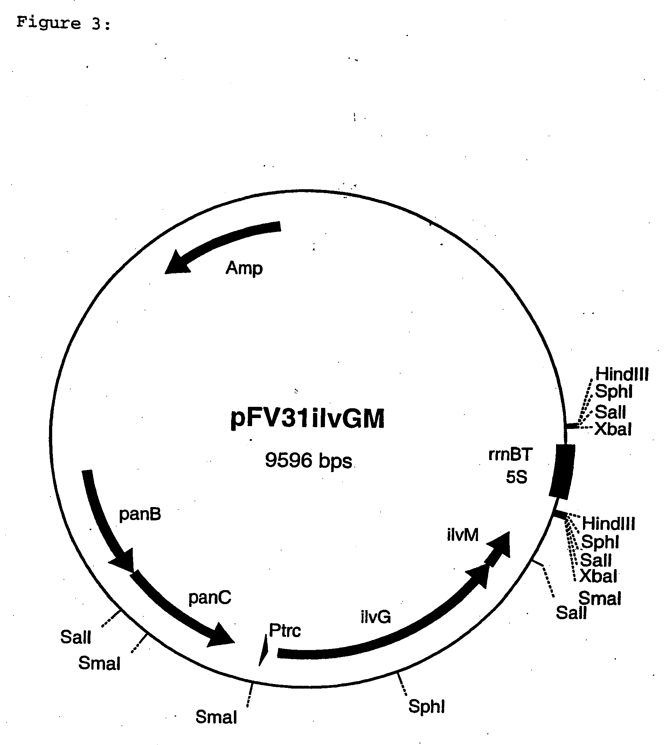

The D-pantothenic acid-producing E. coli strain FV5069/pFV31 is described in EP-A-0 590 857 and is deposited as FERM BP 4395 under the terms of the Budapest Treaty. Plasmid pFV31 is isolated from FV5069/pFV31 and cleaved with the enzyme BamHI and the protruding 3' ends are treated with the Klenow enzyme. This is followed by an alkaline phosphatase treatment. After restriction with the enzyme SspI and separation of the cleavage mixture in 0.8% agarose gel, the 2.8 kbp ilvGM expression cassette is isolated from vector pTrc99AilvGM described in Example 4.2 and ligated with linearized and dephosphorylated vector pFV31. The ligation mixture is transformed into the E. coli strain DH5α and plasmid-carrying cells are selected on LB agar to which 50 µg/ml of ampicillin have been added. The successful cloning of the ilvGM expression cassette can be detected after isolation of the plasmid DNA and control cleavage with HindIII, SalI, SmaI, SphI and XbaI. The plasmid is called pFV31i1vGM (Figure 3).

### 4.4 Preparation of the strain FE6-1ΔyjfA/pFV31ilvGM

The strain FE6-1AyjfA obtained in Example 3 and the strain FE6-1 are transformed with plasmid pFV31ilvGM and transformants are selected on LB agar supplemented with 50 µg/ml of ampicillin. The strains FE6-1AyjfA/pFV31ilvGM and FE6-1/pFV31ilvGM are formed in this way.

### 4.5 Preparation of D-pantothenic acid with the strain FE6-1AyjfΔ/pFV31ilvGM

The pantothenate production of the E. coli strains FE6-1/pFV31ilvGM and FE6-1ΔyjfA/pFV31ilvGm is checked in batch cultures of 10 ml contained in 100 ml Erlenmeyer flasks. To do this, 10 ml of a preculture medium of the following composition: 2 g/l of yeast extract, 10 g/l of (NH₄)₂SO₄, 1 g/l of KH₂PO₄, 0.5 g/l of MgSO₄*7H₂O, 15 g/l of CaCO₃, 20 g/l of glucose and 50 µg/ml of ampicillin, are inoculated with a single colony and incubated for 20 hours at 33°C and 200 rpm on an ESR incubator from Kühner AG (Birsfelden, Switzerland). 200 µl aliquots of this preculture are inoculated into 10 ml of production medium (25 g/l of (NH₄)₂SO₄, 2 g/l of KH₂PO₄, 1 g/l of MgSO₄*7H₂O, 0.03 g/l of FeSO₄*7H₂O, 0.018 g/l of MnSO₄·1H₂O, 30 g/l of CaCO₃, 20 g/l of glucose, 20 g/l of β-alanine, 250 mg/l of thiamine) and incubated for 48 hours at 37°C and 200 rpm. After incubation, the optical density (OD) of the culture suspension is determined with an LP2W photometer from Dr. Lange (Düsseldorf, Germany) at a wavelength of 660 nm.

The concentration of D-pantothenate formed is then determined in the sterile-filtered culture supernatant by means of the Lactobacillus plantarum ATCC8014 pantothenate assay according to DIFCO's instructions (DIFCO MANUAL, 10th edition, pp 1100 - 1102; Michigan, USA). Calibration is effected using calcium D(+)-pantothenate hydrate (catalogue number 25,972-1, Sigma-Aldrich, Deisenhofen, Germany).

The experimental results are shown in Table 1.

**Table 1**

| Strain | OD (660 nm) | Pantothenate g/l |
|---|---|---|
| FE6-1/pFV31ilvGM | 11.1 | 0.78 |
| FE6-1ΔyjfA/ pFV31ilvGM | 10.0 | 1.02 |

### Brief Description of the Figures:

- Figure 1: pMAK705DyjfA (= pMAK705deltayjfA)
- Figure 2: pTrc99AilvGM
- Figure 3: pFV31ilvGM

Length data are to be understood as approximate. The abbreviations and symbols used have the following meanings:
- cat: chloramphenicol resistance gene
- rep-ts: temperature-sensitive replication region of plasmid pSC101
- ytfP'-yjfA': DNA sequence containing truncated coding regions of ytfP and yjfA
- Amp: ampicillin resistance gene
- lacI: gene for the repressor protein of the trc promoter
- Ptrc: trc promoter region, IPTG-inducible
- ilvG: coding region of the large subunit of acetohydroxy acid synthase II
- ilvm: coding region of the small subunit of acetohydroxy acid synthase II
- 5S: 5S rRNA region
- rrnBT: rRNA terminator region
- panB: coding region of the panB gene
- panC: coding region of the panC gene

The abbreviations for the restriction enzymes have the following meanings:
- EcoRI: restriction endonuclease from Escherichia coli
- HindIII: restriction endonuclease from Haemophilus influenzae
- NsiI: restriction endonuclease from Neisseria sicca
- SacI: restriction endonuclease from Streptomyces achromogenes
- SalI: restriction endonuclease from Streptomyces albus
- SmaI: restriction endonuclease from Serratia marcescens
- SphI: restriction endonuclease from Streptomyces phaeochromogenes
- SspI: restriction endonuclease from Sphaerotilus species
- XbaI: restriction endonuclease from Xanthomonas badrii

## Claims

1. Process for the preparation of D-pantothenic acid and/or its alkaline earth metal salts or its salts by the fermentation of genetically modified microorganisms of the family Enterobacteriaceae, whereby the activity or concentration of the proteins encoded by the ytfP open reading frame having the nucleotide sequence of nucleotides 376 to 714 of SEQ ID NO:1 and the yjfA open reading frame having the nucleotide sequence of nucleotides 727 to 461 of the complement of SEQ ID NO:1 is reduced to 0 % of the activity or concentration of the corresponding proteins in the starting microorganisms.

2. Process according to Claim 1, whereby said microorganisms genetically modified in the ytfP ORF and yjfA ORF are achieved by a mutation process chosen from the group transition, transversion, insertion and deletion.

3. Process according to Claim 1 wherein
a) the D-pantothenic acid and/or its salts are enriched in the medium or in the cells of the microorganisms, and
b) when the fermentation has finished, the D-pantothenic acid and/or its salts are isolated, the biomass and/or other constituents of the fermentation broth being separated off in an amount of ≥0 to 100%.

4. Process according to Claim 1, wherein the fermentation is carried out in the presence of alkaline earth metal salts introduced continuously or batchwise in the desired amount, and a product containing or consisting of an alkali metal salt of D-pantothenic acid is recovered.

5. Process according to Claim 1, wherein microorganisms of the genus Escherichia are used.

6. Process according to Claim 4, wherein the microorganisms of the genus Escherichia are derived from the species Escherichia coli.

7. Process according to Claim 1 which comprises amplifying one or more endogenous genes selected from the group comprising:
a) the ilvGM operon coding for acetohydroxy acid synthase II,
b) the panB gene coding for ketopantoate hydroxymethyltransferase,
c) the panE gene coding for ketopantoate reductase,
d) the panD gene coding for aspartate decarboxylase,
e) the panC gene coding for pantothenate synthetase,
f) the serC gene coding for phosphoserine transaminase,
g) the gcvT, gcvH and gcvP genes coding for the glycine cleavage system, and
h) the glyA gene coding for serine hydroxymethyltransferase.

8. Process according to Claim 1 whereby the activity or concentration of the proteins encoded by one or more endogenous genes selected from the group comprising:
a) the avtA gene coding for transaminase C,
b) the poxB gene coding for pyruvate oxidase, and
c) the pckA gene coding for PEP carboxykinase is (are) reduced to 0 % of the activity or concentration of the corresponding proteins in the starting microorganisms.

9. Genetically modified microorganisms of the family Enterobacteriaceae, wherein the activity or concentration of the proteins encoded by the ytfP open reading frame having the nucleotide sequence of nucleotides 376 to 714 of SEQ ID NO:1 and the yjfA open reading frame having the nucleotide sequence of nucleotides 727 to 461 of the complement of SEQ ID NO:1 is reduced to 0 % of the activity or concentration of the corresponding proteins in the starting microorganisms.

10. Microorganisms of according to claim 9, wherein said microrganism are of the species Escherichia coli.

11. Process for the preparation of D-pantothenic acid and/or its salts by the fermentation of microorganisms according to Claim 9 or 10.

12. Process for the preparation of animal feed additives according to claim 1, wherein
a) > 0 to 100 % of the biomass is removed from the fermentation broth, obtained by fermentation and containing D-pantothenic acid and/or its salts,
b) the resulting mixture is optionally concentrated,
c) the mixture containing pantothenic acid and/or pantothenate is converted to a flowable form by suitable means, and
d) a flowable product with a particle size distribution of 20 to 2000 µm, especially of 100 to 1400 µm is prepared.

13. Process according to Claim 12 with a content of D-pantothenic acid and/or its salts selected from the group comprising the magnesium and calcium salts, wherein
a) water is optionally removed from the fermentation broth (concentration),
b) the biomass formed during the fermentation is separated off in an amount of ≥0 to 100%,
c) D-pantothenic acid and/or its salts are optionally added to the fermentation broths treated according to a) and b), the amount of the added compounds being such that their total concentration in the animal feed additive ranges from about 20 to 80 wt.% (dry weight), and
d) the product is produced in the desired pulverulent or, preferably, granular form.

14. Process according to Claim 13, wherein a product is obtained in the desired pulverulent or granular form from the fermentation broth by
a) drying and compaction, or
b) spray-drying, or
c) spray-drying and granulation, or
d) spray-drying and pelletizing,
optionally after the addition of D-pantothenic acid and/or its salts and optionally after the addition of organic or inorganic auxiliary substances.

## Patentansprüche

1. Verfahren zur Herstellung von D-Pantothensäure und/oder ihrer Erdalkalimetallsalze oder ihrer Salze durch Fermentierung genetisch modifizierter Mikroorganismen der Familie Enterobacteriaceae, wobei die Aktivität oder Konzentration der Proteine, die durch das offene ytfP-Leseraster, das die Nukleotidsequenz der Nukleotide 376 bis 714 von SEQ ID NO: 1 aufweist, und das offene yjfA-Leseraster, das die Nukleotidsequenz der Nukleotide 727 bis 461 des Komplements von SEQ ID NO: 1 aufweist, kodiert sind, auf 0 % der Aktivität oder Konzentration der entsprechenden Proteine in den Startmikroorganismen reduziert wird.

2. Verfahren nach Anspruch 1, wobei die Mikroorganismen, die in dem ytfP ORF und yjfA ORF genetisch modifiziert sind, durch einen Mutationsvorgang erhalten werden, ausgewählt aus der Gruppe der Transversion, Insertion und Deletion.

3. Verfahren nach Anspruch 1, wobei
a) die D-Pantothensäure und/oder ihre Salze im Medium oder in den Zellen der Mikroorganismen angereichert werden und
b) wenn die Fermentation abgeschlossen ist, die D-Pantothensäure und/oder ihre Salze isoliert werden, wobei die Biomasse und/oder andere Bestandteile der Fermentationsbrühe in einer Menge von ≥ 0 bis 100 % abgetrennt werden.

4. Verfahren nach Anspruch 1, wobei die Fermentation in Gegenwart von Erdalkalimetallsalzen durchgeführt wird, die in der erwünschten Menge kontinuierlich oder chargenweise zugesetzt werden, und ein Produkt erhalten wird, das ein Alkalimetallsalz der D-Pantothensäure enthält oder daraus besteht.

5. Verfahren nach Anspruch 1, wobei Mikroorganismen der Gattung Escherichia verwendet werden.

6. Verfahren nach Anspruch 4, wobei die Mikroorganismen der Gattung Escherichia aus der Spezies Escherichia coli abgeleitet werden.

7. Verfahren nach Anspruch 1, das das Verstärken eines oder mehrerer endogener Gene umfasst, ausgewählt aus der Gruppe umfassend
a) das ilvGM-Operon, das für Acetohydroxysäuresynthase II kodiert,
b) das panB-Gen, das für Ketopantoathydroxymethyltransferase kodiert
c) das panE-Gen, das für Ketopantoatreduktase kodiert,
d) das panD-Gen, das für Aspartatdecarboxylase kodiert,
e) das panC-Gen, das für Pantothenatsynthetase kodiert,
f) das serC-Gen, das für Phosphoserintransaminase kodiert,
g) die gevT-, gevH- und gcvP-Gene, die für das Glycinspaltungssystem kodieren und
h) das glyA-gen, das für Serinhydroxymethyltransferase kodiert.

8. Verfahren nach Anspruch 1, wobei die Aktivität oder Konzentration der Proteine, die durch eines oder mehrere endogene Gene kodiert werden, ausgewählt aus der Gruppe, umfassend:
a) das avtA-Gen, das für Transaminase C kodiert,
b) das poxB-Gen, das für Pyruvatoxidase kodiert und
c) das pckA-Gen, das für PEP-carboxykinase kodiert
auf 0 % der Aktivität oder Konzentration der entsprechenden Proteine in den Startmikroorganismen reduziert wird.

9. Genetisch modifizierte Mikroorganismen der Familie Enterobacteriaceae, wobei die Aktivität oder Konzentration der Proteine, die durch das offene ytfP-Leseraster, das die Nukleotidsequenz der Nukleotide 376 bis 714 der SEQ ID NO: 1 aufweist, und das offene yjfA-Leseraster, das die Nukleotidsequenz der Nukleotide 727 bis 461 des Komplements von SEQ ID NO: 1 aufweist, kodiert sind, auf 0 % der Aktivität oder Konzentration der entsprechenden Proteine in den Startmikroorganismen reduziert wird.

10. Mikroorganismen nach Anspruch 9, wobei der Mikroorganismus aus der Spezies Escherichia coli stammt.

11. Verfahren zur Herstellung von D-Pantothensäure und/oder ihrer Salze durch Fermentation der Mikroorganismen nach Anspruch 9 oder 10.

12. Verfahren für die Herstellung von Tierfutterzusatzmitteln nach Anspruch 1, wobei
a) >0 bis 100 % der Biomasse aus der Fermentationsbrühe entfernt werden, die durch Fermentieren erhalten wird und D-Pantothensäure und/oder ihre Salze enthält,
b) die dabei entstehende Mischung wahlweise konzentriert wird,
c) die Mischung, die die Pantothensäure und/oder Pantothenat enthält, durch geeignete Mittel in eine fließfähige Form umgewandelt wird, und
d) ein fließfähiges Produkt mit einer Teilchengrößenverteilung von 20 bis 2000 µm, insbesondere 100 bis 1400 µm zubereitet wird.

13. Verfahren nach Anspruch 12 mit einem Gehalt an D-Pantothensäure und/oder seinen Salzen ausgewählt aus der Gruppe umfassend die Magnesium- und Calciumsalze, wobei
a) Wasser wahlweise aus der Fermentationsbrühe entfernt wird (Konzentration),
b) die während des Fermentierens gebildete Biomasse in einer Menge von ≥0 bis 100 % abgetrennt wird,
c) D-Pantothensäure und/oder ihre Salze wahlweise den Fermentationsbrühen zugegeben werden, die a) und b) entsprechend behandelt werden, wobei die Menge an zugesetzten Verbindungen derart ist, dass ihre Gesamtkonzentration im Tierfutterzusatzmittel im Bereich von ca. 20 bis 80 Gew.-% (Trockengewicht) liegt und
d) das Produkt in der erwünschten pulverförmigen Form oder bevorzugt in Granulatform hergestellt wird.

14. Verfahren nach Anspruch 13, wobei ein Produkt in der erwünschten pulverförmigen Form oder Granulatform aus der Fermentationsbrühe erhalten wird durch
a) Trocknen und Verdichten oder
b) Sprühtrocknen oder
c) Sprühtrocknen und Granulieren oder
d) Sprühtrocknen und Pelletisieren,
wahlweise nach Zusetzen von D-Pantothensäure und/oder ihrer Salze und wahlweise nach Zusetzen organischer oder anorganischer Hilfssubstanzen.

## Revendications

1. Procédé pour la production d'acide D-pantothénique et/ou de ses sels de métaux alcalino-terreux ou de ses sels, par culture par fermentation de micro-organismes génétiquement modifiés de la famille des entérobactériacées, par lequel l'activité ou la concentration des protéines codées par le cadre de lecture ouvert (ORF) ytfP ayant la séquence nucléotidique des nucléotides 376 à 714 de SEQ ID n° 1 et le cadre de lecture ouvert yjfA ayant la séquence nucléotidique des nucléotides 727 à 461 du complément de SEQ ID n° 1 est réduite à 0 % de l'activité ou de la concentration des protéines correspondantes chez les micro-organismes de départ.

2. Procédé selon la revendication 1, par lequel lesdits micro-organismes génétiquement modifiés dans l'ORF ytfP et l'ORF yjfA sont obtenus par un processus de mutation choisi dans le groupe constitué par la transition, la transversion, l'insertion et la délétion.

3. Procédé selon la revendication 1, dans lequel
a) la concentration de l'acide D-pantothénique et/ou de ses sels est augmentée dans le milieu ou dans les cellules des micro-organismes, et
b) à la fin de la fermentation on isole l'acide D-pantothénique et/ou ses sels, en séparant la biomasse et/ou les autres constituants du bouillon de fermentation en une proportion de ≥0 à 100 %.

4. Procédé selon la revendication 1, dans lequel on effectue la fermentation en présence de sels de métaux alcalino-terreux introduits en la quantité désirée, en continu ou en mode discontinu, et on récupère un produit contenant ou consistant en un sel de métal alcalin d'acide D-pantothénique.

5. Procédé selon la revendication 1, dans lequel on utilise des micro-organismes appartenant au genre *Escherichia.*

6. Procédé selon la revendication 4, dans lequel les micro-organismes appartenant au genre *Escherichia* sont issus de l'espèce *Escherichia coli.*

7. Procédé selon la revendication 1, comprenant l'amplification d'un ou plusieurs gènes endogènes choisis dans le groupe comprenant :
a) l'opéron ilvGM codant pour l'acétohydroxy acide synthase II,
b) le gène panB codant pour la cétopantoate hydroxyméthyltransférase,
c) le gène panE codant pour la cétopantoate réductase,
d) le gène panD codant pour l'aspartate décarboxylase,
e) le gène panC codant pour la pantothénate synthétase,
f) le gène serC codant pour la phosphosérine transaminase,
g) les gènes gcvT, gcvH et gcvP codant pour le système de coupure de glycine, et
h) le gène glyA codant pour la sérine hydroxyméthyltransférase.

8. Procédé selon la revendication 1, par lequel l'activité ou la concentration des protéines codées par un ou plusieurs gènes endogènes choisis dans le groupe comprenant:
a) le gène avtA codant pour la transaminase C,
b) le gène poxB codant pour la pyruvate oxydase, et
c) le gène pckA codant pour la PEP carboxykinase
est(sont) réduite(s) à 0 % de l'activité ou de la concentration des protéines correspondantes chez les micro-organismes de départ.

9. Micro-organismes génétiquement modifiés de la famille des entérobactériacées, dans lesquels l'activité ou la concentration des protéines codées par le cadre de lecture ouvert (ORF) ytfP ayant la séquence nucléotidique des nucléotides 376 à 714 de SEQ ID n° 1 et le cadre de lecture ouvert yjfA ayant la séquence nucléotidique des nucléotides 727 à 461 du complément de SEQ ID n° 1 est réduite à 0 % de l'activité ou de la concentration des protéines correspondantes chez les micro-organismes de départ.

10. Micro-organismes selon la revendication 9, dans lesquels lesdits micro-organismes appartiennent à l'espèce *Escherichia coli.*

11. Procédé pour la production d'acide D-pantothénique et/ou de ses sels par culture par fermentation de micro-organismes selon la revendication 9 ou 10.

12. Procédé pour la préparation d'additifs à des aliments pour animaux selon la revendication, dans lequel
a) on élimine > 0 à 100 % de la biomasse du bouillon de fermentation obtenu par fermentation et contenant de l'acide D-pantothénique et/ou ses sels,
b) éventuellement on concentre le mélange résultant,
c) le mélange contenant de l'acide pantothénique et/ou un pantothénate est converti par un moyen convenable en une forme apte à l'écoulement, et
d) on prépare un produit apte à l'écoulement, ayant une distribution granulométrique de 20 à 2 000 µm, en particulier de 100 à 1 400 µm.

13. Procédé selon la revendication 12, avec une teneur en acide D-pantothénique et/ou ses sels choisis dans le groupe comprenant les sels de calcium et de magnésium, dans lequel
a) éventuellement on élimine de l'eau du bouillon de fermentation (concentration),
b) on sépare en une proportion de ≥0 à 100 % la biomasse formée au cours de la fermentation,
c) éventuellement on ajoute de l'acide D-pantothénique et/ou ses sels aux bouillons de fermentation traités conformément à a) ou b), la quantité des composés ajoutés étant telle que leur concentration totale dans l'additif à aliment pour animaux aille d'environ 20 à 80 % en poids (poids sec), et
d) on met le produit sous la forme désirée, pulvérulente ou, de préférence, granulaire.

14. Procédé selon la revendication 13, dans lequel un produit est obtenu sous la forme désirée, pulvérulente ou granulaire, à partir du bouillon de formentation, par
a) séchage et compactage, ou
b) séchage par atomisation, ou
c) séchage par atomisation et granulation, ou
d) séchage par atomisation et pastillage,
éventuellement après l'addition d'acide D-pantothénique et/ou de ses sels et éventuellement après l'addition de substances auxiliaires organiques ou minérales.


### SEQUENCE LISTING

<110> Degussa AG
<120> Process for the preparation of D-pantothenic acid and/or its salts
<130> 010393 BT
<160> 10
<170> Patent In version 3.1
<210> 1
   <211> 1248
   <212> DNA
   <213> Escherichia coli
<220>
   <221> gene
   <222> (376)..(714)
   <223> ytfP ORF
<220>
   <221> gene
   <222> complement ((46,1) ..(727))
   <223> yjfA ORF
<400> 1
   ggcgatgtcg caacaagctg ccttgtctta tttgctacgt ggacaagggc tggagagcga 60
   tcagagcgac agtgcggcaa tgacctcgat gctgattggt ttgggggttg cgcaaagtgg 120
   ccagattgtg ggtaaaatcg gcgagacgtt tggcgtaagc aatttagcgc tcgacaccca 180
   gggagtaggc gactcctccc aggtagtggt cagcggctat gtattgccag gtctgcaagt 240
   gaaatacggc gtgggtatat ttgactctat agcaacactc acgttacgtt atcgcctgat 300
   gcctaagcta tatctggaag ccgtgtctgg tgtagaccag gcactggatt tgctctatca 360
   gttcgagttt tagcaatgcg aatatttgtc tacggcagtt tacgccacaa acaaggcaac 420
   agtcactgga tgaccaatgc ccagttactg ggcgatttca gtatcgataa ctaccagttg 480
   tatagcctgg gccactatcc aggcgcagtt ccggggaacg gaacggtaca cggtgaagtt 540
   tatcgtattg acaacgccac gctggccgaa cttgatgcct tgcgcaccag gggcggtgaa 600
   tacgcgcgcc agttgattca gacgccgtac gggagtgcat ggatgtacgt ttatcaacga 660
   cccgtcgatg gattaaagct aattgaaagc ggcgactggt tagacaggga taagtaacca 720
   tatgcatacg ccaccttcgg gtggcgttgt tttttgcgag acgactcgca ttctgttttg 780
   taattccctc accttttgct tttctctccg agccgctttc catatctatt aacgcataaa 840
   aaactctgct ggcattcaca aatgcgcagg ggtaaaacgt ttcctgtagc accgtgagtt 900
   atactttgta taacttaagg aggtgcagat gcgtattacc ataaaaagat gggggaacag 960
   tgcaggtatg gtcattccca atatcgtaat gaaagaactt aacttacagc cggggcagag 1020
   cgtggaggcg caagtgagca acaatcaact gattctgaca cccatctcca ggcgctactc 1080
   gcttgatgaa ctgctggcac agtgtgacat gaacgccgcg gaacttagcg agcaggatgt 1140
   ctggggtaaa tccacccctg cgggtgacga aatatggtaa agaaaagtga atttgaacgg 1200
   ggagacattg tgctggttgg ctttgatcca gcaagcggcc atgaacag 1248
<210> 2
   <211> 20
   <212> DNA
   <213> synthetic sequence
<220>
   <221> primer
   <222> (1)..(20)
   <223> ytfP-1
<400> 2
   ggcgatgtcg caacaagctg 20
<210> 3
   <211> 20
   <212> DNA
   <213> synthetic sequence
<220>
   <221> primer
   <222> (1)..(20)
   <223> ytfP-2
<400> 3
   ctgttcatgg ccgcttgctg                                               20
<210> 4
   <211> 911
   <212> DNA
   <213> Escherichia coli
<220>
   <221> misc_feature
   <222> (1)..(911)
   <223> deletion-carrying ytfP-yjfA region
<220>
   <221> misc_feature
   <222> (1)..(383)
   <223> 5' flank of the ytfP-yjfA region
<220>
   <221> misc_feature
   <222> (389)..(911)
   <223> 3' flank of the ytfP-yjfA region
<220>
   <221> misc_feature
   <222> (376)..(378)
   <223> ATG codon of the truncated ytfP ORF
<220>
   <221> misc_feature
   <222> complement ((388)..(390))
   <223> ATG codon of the truncated yjfA ORF
<400> 4
   ggcgatgtcg caacaagctg ccttgtctta tttgctacgt ggacaagggc tggagagcga 60
   tcagagcgac agtgcggcaa tgacctcgat gctgattggt ttgggggttg cgcaaagtgg 120
   ccagattgtg ggtaaaatcg gcgagacgtt tggcgtaagc aatttagcgc tcgacaccca 180
   gggagtaggc gactcctccc aggtagtggt cagcggctat gtattgccag gtctgcaagt 240
   gaaatacggc gtgggtatat ttgactctat agcaacactc acgttacgtt atcgcctgat 300
   gcctaagcta tatctggaag ccgtgtctgg tgtagaccag gcactggatt tgctctatca 360
   gttcgagttt tagcaatgcg aattatgcat acgccacctt cgggtggcgt tgttttttgc 420
   gagacgactc gcattctgtt ttgtaattcc ctcacctttt gcttttctct ccgagccgct 480
   ttccatatct attaacgcat aaaaaactct gctggcattc acaaatgcgc aggggtaaaa 540
   cgtttcctgt agcaccgtga gttatacttt gtataactta aggaggtgca gatgcgtatt 600
   accataaaaa gatgggggaa cagtgcaggt atggtcattc ccaatatcgt aatgaaagaa 660
   cttaacttac agccggggca gagcgtggag gcgcaagtga gcaacaatca actgattctg 720
   acacccatct ccaggcgcta ctcgcttgat gaactgctgg cacagtgtga catgaacgcc 780
   gcggaactta gcgagcagga tgtctggggt aaatccaccc ctgcgggtga cgaaatatgg 840
   taaagaaaag tgaatttgaa cggggagaca ttgtgctggt tggctttgat ccagcaagcg 900
   gccatgaaca g 911
<210> 5
   <211> 21
   <212> DNA
   <213> synthetic sequence
<220>
   <221> primer
   <222> (1)..(21)
   <223> ilvGM1
<400> 5
   caggacgagg aactaactat g 21
<210> 6
   <211> 20
   <212> DNA
   <213> synthetic sequence
<220>
   <221> primer
   <222> (1)..(20)
   <223> ilvGM2
<400> 6
   tcacgatggc ggaatacaac 20
<210> 7
   <211> 2111
   <212> DNA
   <213> Escherichia coli
<220>
   <221> RBS
<222> (8)..(12)
   <223>
<220>
   <221> mutation
   <222> (8)..(8)
   <223> insertion of the base A in position 8
<220>
   <221> CDS
   <222> (19)..(1665)
   <223> ilvG allele
<400> 7
   caggacgagg aactaact atg aat ggc gca cag tgg gtg gta cat gcg ttg      51
                       Met Asn Gly Ala Gln Trp Val Val His Ala Leu
                       1               5                   10
   cgg gca cag ggt gtg aac acc gtt ttc ggt tat ccg ggt ggc gca att      99
   Arg Ala Gln Gly Val Asn Thr Val Phe Gly Tyr Pro Gly Gly Ala Ile
               15                  20                  25
   atg ccg gtt tac gat gca ttg tat gac ggc ggc gtg gag cac ttg ctg     147
   Met Pro Val Tyr Asp Ala Leu Tyr Asp Gly Gly Val Glu His Leu Leu
           30                  35                  40
   tgc cga cat gag cag ggt gcg gca atg gcg gct atc ggt tat gcc cgt     195
   Cys Arg His Glu Gln Gly Ala Ala Met Ala Ala Ile Gly Tyr Ala Arg
       45                  50                  55
   gct acc ggc aaa act ggc gta tgt atc gcc acg tct ggt ccg ggc gca     243
   Ala Thr Gly Lys Thr Gly Val Cys Ile Ala Thr Ser Gly Pro Gly Ala
   60                  65                  70                  75
   acc aac ctg ata acc ggg ctt gcg gac gca ctg tta gat tct atc cct     291
   Thr Asn Leu Ile Thr Gly Leu Ala Asp Ala Leu Leu Asp Ser Ile Pro
                   80                  85                  90
   gtt gtt gcc atc acc ggt caa gtg tcc gca ccg ttt atc ggc acg gac     339
   Val Val Ala Ile Thr Gly Gln Val Ser Ala Pro Phe Ile Gly Thr Asp
               95                  100                 105
   gca ttt cag gaa gtg gat gtc ctg gga ttg tcg tta gcc tgt acc aag     387
   Ala Phe Gln Glu Val Asp Val Leu Gly Leu Ser Leu Ala Cys Thr Lys
           110                 115                 120
   cac agc ttt ctg gtg cag tcg ctg gaa gag ttg ccg cgc att atg gct     435
   His Ser Phe Leu Val Gln Ser Leu Glu Glu Leu Pro Arg Ile Met Ala
       125                 130                 135
   gaa gca ttc gac gtt gcc agc tca ggt cgt cct ggt ccg gtt ctg gtc     483
   Glu Ala Phe Asp Val Ala Ser Ser Gly Arg Pro Gly Pro Val Leu Val
   140                 145                 150                 155
   gat atc cca aaa gat atc cag cta gcc agc ggt gac ctg gaa ccg tgg     531
   Asp Ile Pro Lys Asp Ile Gln Leu Ala Ser Gly Asp Leu Glu Pro Trp
                   160                 165                 170
   ttc acc acc gtt gaa aac gaa gtg act ttc cca cat gcc gaa gtt gag     579
   Phe Thr Thr Val Glu Asn Glu Val Thr Phe Pro His Ala Glu Val Glu
               175                 180                 185
   caa gcg cgc cag atg ctg gca aaa gcg caa aaa ccg atg ctg tac gtt     627
   Gln Ala Arg Gln Met Leu Ala Lys Ala Gln Lys Pro Met Leu Tyr Val
           190                 195                 200
   ggt ggt ggc gtg ggt atg gcg cag gca gtt cct gct tta cga gaa ttt     675
   Gly Gly Gly Val Gly Met Ala Gln Ala Val Pro Ala Leu Arg Glu Phe
       205                 210                 215
   ctc gct acc aca aaa atg cct gcc acc tgc acg ctg aaa ggg ctg ggc     723
   Leu Ala Thr Thr Lys Met Pro Ala Thr Cys Thr Leu Lys Gly Leu Gly
   220                 225                 230                 235
   gca gtt gaa gca gat tat ccg tac tat ctg ggc atg ctg gga atg cat     771
   Ala Val Glu Ala Asp Tyr Pro Tyr Tyr Leu Gly Met Leu Gly Met His
                   240                 245                 250
   ggc acc aaa gcg gcg aac ttc gcg gtg cag gag tgc gac ttg ctg atc     819
   Gly Thr Lys Ala Ala Asn Phe Ala Val Gln Glu Cys Asp Leu Leu Ile
               255                 260                 265
   gcc gtg ggt gca cgt ttt gat gac cgg gtg acc ggc aaa ctg aac acc     867
   Ala Val Gly Ala Arg Phe Asp Asp Arg Val Thr Gly Lys Leu Asn Thr
           270                 275                 280
   ttc gca cca cac gcc agt gtt atc cat atg gat atc gac ccg gca gaa     915
   Phe Ala Pro His Ala Ser Val Ile His Met Asp Ile Asp Pro Ala Glu
       285                 290                 295
   atg aac aag ctg cgt cag gca cat gtg gca tta caa ggt gat tta aat     963
   Met Asn Lys Leu Arg Gln Ala His Val Ala Leu Gln Gly Asp Leu Asn
   300                 305                 310                 315
   gct ctg tta cca gca tta cag cag ccg tta aat atc aat gac tgg cag    1011
   Ala Leu Leu Pro Ala Leu Gln Gln Pro Leu Asn Ile Asn Asp Trp Gln
                   320                 325                 330
   cta cac tgc gcg cag ctg cgt gat gaa cat gcc tgg cgt tac gac cat    1059
   Leu His Cys Ala Gln Leu Arg Asp Glu His Ala Trp Arg Tyr Asp His
               335                 340                 345
   ccc ggt gac gct atc tac gcg cca ttg ttg tta aaa caa ctg tcg gat    1107
   Pro Gly Asp Ala Ile Tyr Ala Pro Leu Leu Leu Lys Gln Leu Ser Asp
           350                 355                 360
   cgt aaa cct gcg gat tgc gtc gtg acc aca gat gtg ggg cag cac cag    1155
   Arg Lys Pro Ala Asp Cys Val Val Thr Thr Asp Val Gly Gln His Gln
       365                 370                 375
   atg tgg gcc gcg cag cac atc gca cac act cgc ccg gaa aat ttc att    1203
   Met Trp Ala Ala Gln His Ile Ala His Thr Arg Pro Glu Asn Phe Ile
   380                 385                 390                 395
   acc tcc agc ggc tta ggc acc atg ggt ttc ggt tta cca gcg gcg gtt    1251
   Thr Ser Ser Gly Leu Gly Thr Met Gly Phe Gly Leu Pro Ala Ala Val
                   400                 405                 410
   ggc gca caa gtc gca cga ccg aac gat act gtc gtc tgt atc tcc ggt    1299
   Gly Ala Gln Val Ala Arg Pro Asn Asp Thr Val Val Cys Ile Ser Gly
               415                 420                 425
   gac ggc tct ttc atg atg aat gtg caa gag ctg ggc acc gta aaa cgc    1347
   Asp Gly Ser Phe Met Met Asn Val Gln Glu Leu Gly Thr Val Lys Arg
           430                 435                 440
   aag cag tta ccg ttg aaa atc gtc tta ctc gat aac caa cgg tta ggg    1395
   Lys Gln Leu Pro Leu Lys Ile Val Leu Leu Asp Asn Gln Arg Leu Gly
       445                 450                 455
   atg gtt cga caa tgg cag caa ctg ttt ttt cag gaa cga tac agc gaa     1443
   Met Val Arg Gln Trp Gln Gln Leu Phe Phe Gln Glu Arg Tyr Ser Glu
   460                 465                 470                 475
   acc acc ctt act gat aac ccc gat ttc ctc atg tta gcc agc gcc ttc     1491
   Thr Thr Leu Thr Asp Asn Pro Asp Phe Leu Met Leu Ala Ser Ala Phe
                   480                 485                 490
   ggc atc cct ggc caa cac atc acc cgt aaa gac cag gtt gaa gcg gca     1539
   Gly Ile Pro Gly Gln His Ile Thr Arg Lys Asp Gln Val Glu Ala Ala
               495                 500                 505
   ctc gac acc atg ctg aac agt gat ggg cca tac ctg ctt cat gtc tca     1587
   Leu Asp Thr Met Leu Asn Ser Asp Gly Pro Tyr Leu Leu His Val Ser
           510                 515                 520
   atc gac gaa ctt gag aac gtc tgg ccg ctg gtg ccg cct ggc gcc agt     1635
   Ile Asp Glu Leu Glu Asn Val Trp Pro Leu Val Pro Pro Gly Ala Ser
       525                 530                 535
   aat tca gaa atg ttg gag aaa tta tca tga tgcaacatca ggtcaatgta       1685
   Asn Ser Glu Met Leu Glu Lys Leu Ser
   540                 545
   tcggctcgct tcaatccgga aaccttagaa cgtgttttac gcgtggtgcg tcatcgtggt   1745
   ttccacgtct gctcaatgaa tatggctgcc gccagcgatg cacaaaatat aaatatcgaa   1805
   ttgaccgttg ccagcccacg gtcggtcgac ttactgttta gtcagttaaa taaactggtg   1865
   gacgtcgcac acgttgccat ctgccagagc acaaccacat cacaacaaat ccgcgcctga   1925
   gcgcaaaagg aatataaaaa tgaccacgaa gaaagctgat tacatttggt tcaatgggga   1985
   gatggttcgc tgggaagacg cgaaggtgca tgtgatgtcg cacgcgctgc actatggcac   2045
   ctcggttttt gaaggcatcc gttgctacga ctcacacaaa ggaccggttg tattccgcca   2105
   tcgtga                                                              2111
<210> 8
   <211> 548
   <212> PRT
   <213> Escherichia coli
<400> 8
   Met Asn Gly Ala Gln Trp Val Val His Ala Leu Arg Ala Gln Gly Val
   1               5                   10                  15
   Asn Thr Val Phe Gly Tyr Pro Gly Gly Ala Ile Met Pro Val Tyr Asp
               20                  25                  30
   Ala Leu Tyr Asp Gly Gly Val Glu His Leu Leu Cys Arg His Glu Gln
           35                  40                  45
   Gly Ala Ala Met Ala Ala Ile Gly Tyr Ala Arg Ala Thr Gly Lys Thr
       50                  55                  60
   Gly Val Cys Ile Ala Thr Ser Gly Pro Gly Ala Thr Asn Leu Ile Thr
   65                  70                  75                  80
   Gly Leu Ala Asp Ala Leu Leu Asp Ser Ile Pro Val Val Ala Ile Thr
                   85                  90                  95
   Gly Gln Val Ser Ala Pro Phe Ile Gly Thr Asp Ala Phe Gln Glu Val
               100                 105                 110
   Asp Val Leu Gly Leu Ser Leu Ala Cys Thr Lys His Ser Phe Leu Val
           115                 120                 125
   Gln Ser Leu Glu Glu Leu Pro Arg Ile Met Ala Glu Ala Phe Asp Val
       130                 135                 140
   Ala Ser Ser Gly Arg Pro Gly Pro Val Leu Val Asp Ile Pro Lys Asp
   145                 150                 155                 160
   Ile Gln Leu Ala Ser Gly Asp Leu Glu Pro Trp Phe Thr Thr Val Glu
                   165                 170                 175
   Asn Glu Val Thr Phe Pro His Ala Glu Val Glu Gln Ala Arg Gln Met
               180                 185                 190
   Leu Ala Lys Ala Gln Lys Pro Met Leu Tyr Val Gly Gly Gly Val Gly
           195                 200                 205
   Met Ala Gln Ala Val Pro Ala Leu Arg Glu Phe Leu Ala Thr Thr Lys
       210                 215                 220
   Met Pro Ala Thr Cys Thr Leu Lys Gly Leu Gly Ala Val Glu Ala Asp
   225                 230                 235                 240
   Tyr Pro Tyr Tyr Leu Gly Met Leu Gly Met His Gly Thr Lys Ala Ala
                   245                 250                 255
   Asn Phe Ala Val Gln Glu Cys Asp Leu Leu Ile Ala Val Gly Ala Arg
               260                 265                 270
   Phe Asp Asp Arg Val Thr Gly Lys Leu Asn Thr Phe Ala Pro His Ala
           275                 280                 285
   Ser Val Ile His Met Asp Ile Asp Pro Ala Glu Met Asn Lys Leu Arg
       290                 295                 300
   Gln Ala His Val Ala Leu Gln Gly Asp Leu Asn Ala Leu Leu Pro Ala
   305                 310                 315                 320
   Leu Gln Gln Pro Leu Asn Ile Asn Asp Trp Gln Leu His Cys Ala Gln
                   325                 330                 335
   Leu Arg Asp Glu His Ala Trp Arg Tyr Asp His Pro Gly Asp Ala Ile
               340                 345                 350
   Tyr Ala Pro Leu Leu Leu Lys Gln Leu Ser Asp Arg Lys Pro Ala Asp
           355                 360                 365
   Cys Val Val Thr Thr Asp Val Gly Gln His Gln Met Trp Ala Ala Gln
       370                 375                 380
   His Ile Ala His Thr Arg Pro Glu Asn Phe Ile Thr Ser Ser Gly Leu
   385                 390                 395                 400
   Gly Thr Met Gly Phe Gly Leu Pro Ala Ala Val Gly Ala Gln Val Ala
                   405                 410                 415
   Arg Pro Asn Asp Thr Val Val Cys Ile Ser Gly Asp Gly Ser Phe Met
               420                 425                 430
   Met Asn Val Gln Glu Leu Gly Thr Val Lys Arg Lys Gln Leu Pro Leu
   435 440 445
   Lys Ile Val Leu Leu Asp Asn Gln Arg Leu Gly Met Val Arg Gln Trp
   450 455 460
   Gln Gln Leu Phe Phe Gln Glu Arg Tyr Ser Glu Thr Thr Leu Thr Asp
   465 470 475 480
   Asn Pro Asp Phe Leu Met Leu Ala Ser Ala Phe Gly Ile Pro Gly Gln
   485 490 495
   His Ile Thr Arg Lys Asp Gln Val Glu Ala Ala Leu Asp Thr Met Leu
   500 505 510
   Asn Ser Asp Gly Pro Tyr Leu Leu His Val Ser Ile Asp Glu Leu Glu
   515 520 525
   Asn Val Trp Pro Leu Val Pro Pro Gly Ala Ser Asn Ser Glu Met Leu
   530 535 540
   Glu Lys Leu Ser
   545
<210> 9
   <211> 2111
   <212> DNA
   <213> Escherichia coli
<220>
   <221> RBS
   <222> (8)..(12)
   <223>
<220>
   <221> mutation
   <222> (8)..(8)
   <223> insertion of the base A in position 8
<220>
   <221> CDS
   <222> (1662)..(1925)
   <223> ilvM allele
<400> 9
   caggacgagg aactaactat gaatggcgca cagtgggtgg tacatgcgtt gcgggcacag 60
   ggtgtgaaca ccgttttcgg ttatccgggt ggcgcaatta tgccggttta cgatgcattg 120
   tatgacggcg gcgtggagca cttgctgtgc cgacatgagc agggtgcggc aatggcggct 180
   atcggttatg cccgtgctac cggcaaaact ggcgtatgta tcgccacgtc tggtccgggc 240
   gcaaccaacc tgataaccgg gcttgcggac gcactgttag attctatccc tgttgttgcc 300
   atcaccggtc aagtgtccgc accgtttatc ggcacggacg catttcagga agtggatgtc 360
   ctgggattgt cgttagcctg taccaagcac agctttctgg tgcagtcgct ggaagagttg 420
   ccgcgcatta tggctgaagc attcgacgtt gccagctcag gtcgtcctgg tccggttctg 480
   gtcgatatcc caaaagatat ccagctagcc agcggtgacc tggaaccgtg gttcaccacc 540
   gttgaaaacg aagtgacttt cccacatgcc gaagttgagc aagcgcgcca gatgctggca 600
   aaagcgcaaa aaccgatgct gtacgttggt ggtggcgtgg gtatggcgca ggcagttcct   660
   gctttacgag aatttctcgc taccacaaaa atgcctgcca cctgcacgct gaaagggctg   720
   ggcgcagttg aagcagatta tccgtactat ctgggcatgc tgggaatgca tggcaccaaa   780
   gcggcgaact tcgcggtgca ggagtgcgac ttgctgatcg ccgtgggtgc acgttttgat   840
   gaccgggtga ccggcaaact gaacaccttc gcaccacacg ccagtgttat ccatatggat   900
   atcgacccgg cagaaatgaa caagctgcgt caggcacatg tggcattaca aggtgattta   960
   aatgctctgt taccagcatt acagcagccg ttaaatatca atgactggca gctacactgc  1020
   gcgcagctgc gtgatgaaca tgcctggcgt tacgaccatc ccggtgacgc tatctacgcg  1080
   ccattgttgt taaaacaact gtcggatcgt aaacctgcgg attgcgtcgt gaccacagat  1140
   gtggggcagc accagatgtg ggccgcgcag cacatcgcac acactcgccc ggaaaatttc  1200
   attacctcca gcggcttagg caccatgggt ttcggtttac cagcggcggt tggcgcacaa  1260
   gtcgcacgac cgaacgatac tgtcgtctgt atctccggtg acggctcttt catgatgaat  1320
   gtgcaagagc tgggcaccgt aaaacgcaag cagttaccgt tgaaaatcgt cttactcgat  1380
   aaccaacggt tagggatggt tcgacaatgg cagcaactgt tttttcagga acgatacagc  1440
   gaaaccaccc ttactgataa ccccgatttc ctcatgttag ccagcgcctt cggcatccct  1500
   ggccaacaca tcacccgtaa agaccaggtt gaagcggcac tcgacaccat gctgaacagt  1560
   gatgggccat acctgcttca tgtctcaatc gacgaacttg agaacgtctg gccgctggtg  1620
   ccgcctggcg ccagtaattc agaaatgttg gagaaattat c atg atg caa cat cag  1676
                                                 Met Met Gln His Gln
                                                 1               5
   gtc aat gta tcg gct cgc ttc aat ccg gaa acc tta gaa cgt gtt tta    1724
   Val Asn Val Ser Ala Arg Phe Asn Pro Glu Thr Leu Glu Arg Val Leu
                   10                  15                  20
   cgc gtg gtg cgt cat cgt ggt ttc cac gtc tgc tca atg aat atg gct    1772
   Arg Val Val Arg His Arg Gly Phe His Val Cys Ser Met Asn Met Ala
               25                  30                  35
   gcc gcc agc gat gca caa aat ata aat atc gaa ttg acc gtt gcc agc    1820
   Ala Ala Ser Asp Ala Gln Asn Ile Asn Ile Glu Leu Thr Val Ala Ser
           40                  45                  50
   cca cgg tcg gtc gac tta ctg ttt agt cag tta aat aaa ctg gtg gac    1868
   Pro Arg Ser Val Asp Leu Leu Phe Ser Gln Leu Asn Lys Leu Val Asp
       55                  60                  65
   gtc gca cac gtt gcc atc tgc cag agc aca acc aca tca caa caa atc    1916
   Val Ala His Val Ala Ile Cys Gln Ser Thr Thr Thr Ser Gln Gln Ile
   70                  75                  80                  85
   cgc gcc tga gcgcaaaagg aatataaaaa tgaccacgaa gaaagctgat             1965
   Arg Ala
   tacatttggt tcaatgggga gatggttcgc tgggaagacg cgaaggtgca tgtgatgtcg  2025
   cacgcgctgc actatggcac ctcggttttt gaaggcatcc gttgctacga ctcacacaaa  2085
   ggaccggttg tattccgcca tcgtga 2111
<210> 10
   <211> 87
   <212> PRT
   <213> Escherichia coli
<400> 10
   Met Met Gln His Gln Val Asn Val Ser Ala Arg Phe Asn Pro Glu Thr
   1               5                   10                  15
   Leu Glu Arg Val Leu Arg Val Val Arg His Arg Gly Phe His Val Cys
               20                  25                  30
   Ser Met Asn Met Ala Ala Ala Ser Asp Ala Gln Asn Ile Asn Ile Glu
           35                  40                  45
   Leu Thr Val Ala Ser Pro Arg Ser Val Asp Leu Leu Phe Ser Gln Leu
       50                  55                  60
   Asn Lys Leu Val Asp Val Ala His Val Ala Ile Cys Gln Ser Thr Thr
   65                  70                  75                  80
   Thr Ser Gln Gln Ile Arg Ala
                   85